# EUROPEAN PATENT APPLICATION

(11) **EP 4 134 099 A1**
(43) Date of publication of application: **15.02.2023**
(21) Application number: 21785654.1
(22) Date of filing: 06.04.2021
(51) Int. Cl.: A61K 45/00, A61K 31/409, A61K 38/16, A61K 39/395, A61K 41/00, A61K 47/68, A61P 35/00

(54) **MEDICINE FOR KILLING TUMOR CELLS**

(30) Priority: 06.04.2020 JP 2020068071
(71) Applicant: PhotoQ3 Inc., Tokyo 102-0084 (JP)
(72) Inventor: TODA, Naoko, Tokyo 102-0084 (JP); SUDO, Yukio, Tokyo 102-0084 (JP); HAMAKUBO, Takao, Tokyo 102-0084 (JP)
(74) Representative: Hoffmann Eitle
(86) International application number: PCT/JP2021/014588
(87) International publication number: WO 2021/206078

(57) **Abstract**

It is an object of the present invention to provide a medicament for killing tumor cells, having few side effects. According to the present invention, provided is a medicament for killing tumor cells that express a target substance at a low level on the cell surface thereof, said medicament comprising: a conjugate of a substance that binds to a target substance on the surface of tumor cells and a cytotoxin; and a photosensitizing dye.

## Description

### Technical Field

The present invention relates to a medicament for killing tumor cells that express a target substance at a low level on the cell surface thereof, said medicament comprising a conjugate of a substance that binds to a target substance on the surface of tumor cells and a cytotoxin, and a photosensitizing dye.

### Background Art

In Japan, approximately 370,000 people die of cancer each year, and cancer has continuously been a leading cause of death since 1981. To date, a large number of therapeutic methods for cancer have been developed inside and outside of the country. However, a specific remedy for cancer has not yet been developed.

What is called, small molecule anticancer agents (cancer chemotherapy) have been developed. However, the medicinal effects of these anticancer agents are not strong enough, and further, severe side effects cause trouble to patients. Thus, it cannot be said that these anticancer agents are desirable medicaments. Since antibody drugs are characterized in that the antibody drugs have strong specificity to cancer, strong side effects found in the small molecule anticancer agents can be alleviated, and thus, such antibody drugs can be broadly used. However, there are yet only a small number of antibody drugs effective for solid cancer. In order to reinforce medicinal effects, ADC (antibody-drug conjugate) has been developed, but such ADC has not yet been satisfactory, including its toxicity.

Moreover, an immunotherapeutic antibody (checkpoint inhibitor) used as a novel antibody drug exhibits strong medicinal effects against a wide range of cancer species based on its novel mechanism. However, it has been found that the number of patients who receive the effects of such an immunotherapeutic antibody is not necessarily large, and that, in some cases, the patients have severe side effects to such an extent that they lead to death.

However, antibody drugs including ADC have another essential disadvantage that effects cannot be obtained if the number of antigens expressed on the tumor cell surface, to which antibodies bind, is small. It is considered that, in general, anticipated medicinal effects cannot be exhibited, unless the number of target substances to which antibodies bind is, at least, approximately 100,000 molecules per cell.

The expression level of a target antigen in tumor tissues is not necessarily uniform among tumor cells, and antibody drugs cannot exhibit effects on tumor cells expressing such a target antigen at a low level. In addition, the number of expressed targets is different depending on patients, and antibody drugs can exhibit effects only on some patients having a high expression level of target antigen. These essential disadvantages result in a lower response rate than expected.

As another approach, based on the idea that a therapeutic site is restricted and side effects are thereby alleviated, a method of destroying tumor cells, comprising using a combination of a photosensitizing dye which tends to localize in an endosomal membrane and an immunotoxin, applying a light to the photosensitizing dye so as to release the immunotoxin (or a fragment thereof) that has been incorporated into the endosome into the cytoplasm, and thereby destroying the tumor cells, has been conceived. However, to date, if the target on the surface of tumor cells, to which the immunotoxin binds, has not been expressed at a high level, effects could not be found. For example, even in a case where the target on the surface of tumor cells is EGFR, the cases of the expression of medicinal effects have been reported only in cell lines in which the target is highly expressed (Non-Patent Document 1). As in the case of ordinary antibody drugs, it is considered that the aforementioned combination of a photosensitizing dye and an immunotoxin does not have effects on tumor cells that express a target at a low level on the surface thereof.

In view of the foregoing, when an antibody has been used to kill tumor cells, the high expression of a target substance on the surface of the tumor cells has been an essential requirement. On the other hand, in clinical sites, the expression level of a target substance on the surface of tumor cells has often not been uniform among patients, in a single patient (a primary site or a metastatic site thereof), and in cancer tissues, and as a result, this has caused a problem that the range of therapeutic effects has been narrowed.

### Prior Art Documents

### Non-Patent Documents

Non-Patent Document 1: Targeted Delivery and Enhanced Cytotoxicity of Cetuximab-Saporin by Photochemical Internalization in EGFR-Positive Cancer Cells: Wai Lam Yip,† Anette Weyergang,† Kristian Berg,† Hanne H. Tønnesen,‡ and Pål K. Selbo∗,† MOLECULAR PHARMACEUTICS 2007 (VOL. 4), NO. 2, 241-251

### Summary of Invention

### Object to be Solved by the Invention

Under such circumstances, the present inventors have felt a need to develop a pharmaceutical product having few side effects and strong medicinal effects against tumor cells that express a target antigen at a low level, and have thereby achieved the present development goals. It is an object of the present invention to provide a medicament for killing tumor cells that express a target antigen at a low level, said medicament having few side effects.

### Means for Solving the Object

With regard to a pharmaceutical product used against tumor cells that express a target antigen at a low level, the present inventors have conceived a method of applying a light to a tumor site to exhibit medicinal effects thereon, so as to restrict the range in which medicinal effects are exhibited.

The low expression level of the target antigen in this invention means the expression of 20,000 molecules or less per cell, on which ordinary antibody drugs (including ADC) hardly exhibit medicinal effects.

There has also been conceived a method of destroying tumor cells by a combination of a photosensitizing dye which tends to localize in an endosomal membrane and an immunotoxin. This method is considered to be a method, comprising accumulating a photosensitizing dye in an endosomal membrane, then applying a light to this to destroy the endosomal membrane, and thereby releasing an immunotoxin (or a fragment thereof) that has been incorporated into the endosome into a cytoplasm, so as to destroy the tumor cells. Because of localized light irradiation and the specificity of the immunotoxin binding to the tumor cells, this method is anticipated to provide specific effects.

However, even according to this method, it has been considered that, if the expression level of the target substance on the cell surface, to which the immunotoxin binds, is not high, no effects can be obtained, as mentioned above.

The present inventors have conducted intensive studies directed towards solving the previous problems. As a result, the inventors have found that, by allowing a photosensitizing dye and a conjugate of a substance that binds to a target substance on the surface of tumor cells and a cytotoxin to come into contact with tumor cells, and then adjusting the amount of light applied, the time of light application, etc., cytotoxicity against the tumor cells is generated even in a case where the expression level of the target substance on the surface of the tumor cells is significantly lower than that conventionally recognized, thereby completing the present invention.

According to the present invention, the following inventions are provided.
<1> A medicament for killing tumor cells that express a target substance at a low level on the cell surface thereof, comprising:
   a conjugate of a substance that binds to a target substance on the surface of tumor cells and a cytotoxin; and
   a photosensitizing dye.
<2> The medicament according to <1>, wherein the expression level of the target antigen is 20,000 molecules or less per cell.
<3> The medicament according to <1> or <2>, which is a medicament for killing a tumor cell group comprising tumor cells, in which the expression level of the target substance is 20,000 molecules or less per cell.
<4> The medicament according to any one of <1> to <3>, wherein the substance that binds to the target substance on the surface of the tumor cells is an antibody, an antibody fragment, a ligand, or a peptide.
<5> The medicament according to any one of <1> to <4>, wherein the cytotoxin is saporin, gelonin, or Pseudomonas exotoxin.
<6> The medicament according to any one of <1> to <5>, wherein the photosensitizing dye is Talaporfin Sodium, Porfimer Sodium, AlPcS2a, or TPCS2a.
<7> The medicament according to any one of <1> to <6>, wherein the tumor cells are cells that express Epidermal Growth Factor Receptor (EGFR, ERBB1, ERBB2, ERBB3, or ERBB4), Mesothelin, Ephrin type-A receptor 2 (EphA2), Glypican 3 (GPC3), Cadherin 17 (CDH17), or Roundabout homolog 1 (Robo1) on the cell surface thereof.
<8> The medicament according to any one of <1> to <7>, wherein the tumor cells are cancer cells of any one of head and neck cancer, lung cancer, liver cancer, colorectal cancer, skin cancer, esophageal cancer, stomach cancer, cervical cancer, endometrial cancer, mesothelioma, brain tumor, malignant melanoma, breast cancer, bile duct cancer, pancreatic cancer, ovarian cancer, kidney cancer, bladder cancer, prostate cancer, malignant lymphoma, and osteosarcoma.
<9> The medicament according to any one of <1> to <8>, which kills tumor cells by performing the following steps on the cells:
   (1) a step of allowing the conjugate to come into contact with tumor cells;
   (2) a step of allowing a photosensitizing dye to come into contact with the tumor cells; and
   (3) a step of irradiating the tumor cells with a wavelength effective for activating the photosensitizing dye, so as to kill the cells.
<10> The medicament according to any one of <1> to <9>, which kills tumor cells by performing the following steps on the cells:
   (1) a step of allowing the conjugate and a photosensitizing dye to come into contact with tumor cells; and then,
   (2) a step of irradiating the tumor cells with a wavelength effective for activating the photosensitizing dye, so as to kill the cells.

< A > A method for killing tumor cells that express a target substance at a low level, said method comprising:
   (1) a step of allowing a conjugate of a substance that binds to a target substance expressed at a low level on the surface of tumor cells and a cytotoxin, to come into contact with the tumor cells;
   (2) a step of allowing a photosensitizing dye to come into contact with the tumor cells; and
   (3) a step of irradiating the tumor cells with a wavelength effective for activating the photosensitizing dye, so as to kill the cells.
< B > A method for killing tumor cells that express a target substance at a low level, said method comprising:
   (1) a step of allowing a conjugate of a substance that binds to a target substance expressed at a low level on the surface of tumor cells and a cytotoxin, and a photosensitizing dye, to come into contact with the tumor cells; and
   (2) a step of irradiating the tumor cells with a wavelength effective for activating the photosensitizing dye, so as to kill the cells.

### Advantageous Effects of Invention

According to the present invention, a medicament for killing tumor cells, having few side effects, can be provided.

### Brief Description of Drawings

[Fig. 1] Fig. 1 shows cell viability in a cytotoxicity assay in which aluminum phthalocyanine was used to MCF7 cells.
[Fig. 2] Fig. 2 shows cell viability in a cytotoxicity assay in which Talaporfin Sodium was used to MCF7 cells.

### Embodiments of Carrying out the Invention

Hereinafter, the embodiments of the present invention will be described in detail.

### < Summary of the Invention >

The present inventors have studied a method for treating a tumor that expresses a target antigen at a low level, said method having strong medicinal effects and few side effects. As a result, the inventors have conceived that the technique of combining a photosensitizing dye and an immunotoxin that locally exhibit strong medicinal effects as a result of light irradiation has potential. However, it has not been considered so far that this technique exhibits effects on cells in which the expression level of target cells is low.

Hence, the present inventors have conducted intensive studies, and as a result, they have combined a photosensitizing dye such as Talaporfin Sodium or AlPcS2a, "a conjugate of a substance that binds to a target substance on the surface of tumor cells and a cytotoxin" such as an immunotoxin, and "application of a light to a tumor," so that the inventors have discovered a therapeutic method that exhibits strong medicinal effects even on tumor cells that express the target substance at a low level and has few side effects, thereby completing the present invention.

Specifically, in the present invention, a conjugate of a substance that binds to a target substance on the surface of tumor cells and a cytotoxin binds to a tumor, and it is then enclosed in the endosome. It is conceived that a light is then applied to a photosensitizing dye, which has been added separately (or simultaneously), so that an immunotoxin (or a decomposed product thereof) in the endosome is released into the cytoplasm, and the tumor cells can be thereby killed.

### < Expression level of target antigen on tumor cells >

In the present invention, the low expression level of a target antigen on tumor cells means that the expression level of a target antigen per cell is 20,000 molecules or less. The expression level of a target antigen per cell can be measured by an ordinary method such as flow cytometry or ELISA. Moreover, a technique such as immunostaining or MS proteomics analysis can also be applied.

In the present invention, the medicament is characterized in that it can exhibit effects on tumor cells that express a target antigen at a low level on the surface thereof. Thus, it is obvious that the present medicament can also exhibit effects on tumor cells that express a target antigen at a higher level than the aforementioned tumor cells. Moreover, it can be expected that the present medicament will exhibit effects on a group of tumor cells each having a different expression level.

### < Embodiments regarding Method for Killing Cells >

The method for killing tumor cells of the present invention may include an embodiment in which tumor cells are irradiated with (3) a light having a wavelength for activating a photosensitizing dye in the coexistence of (1) a conjugate of a substance that binds to a target substance on the surface of tumor cells and a cytotoxin, and (2) the photosensitizing dye.

As a further specific embodiment, the killing of tumor cells can also be achieved by administering (1) a conjugate of a substance that binds to a target substance on the surface of tumor cells and a cytotoxin to tumor cells, then administering thereto (2) a photosensitizing dye, and then irradiating the cells with (3) a light having a wavelength for activating the photosensitizing dye.

In addition, as another embodiment, the killing of tumor cells can also be achieved by administering (1) a photosensitizing dye to tumor cells, then administering thereto (2) a conjugate of a substance that binds to a target substance on the surface of tumor cells and a cytotoxin, and then irradiating the cells with (3) a light having a wavelength for activating the photosensitizing dye.

Moreover, as another embodiment, the killing of tumor cells can also be achieved by simultaneously administering (1) a conjugate of a substance that binds to a target substance on the surface of tumor cells and a cytotoxin and (2) a photosensitizing dye to tumor cells, and then irradiating the cells with (3) a light having a wavelength for activating the photosensitizing dye.

### < Photosensitizing dye >

The photosensitizing dye used in the present invention is a sensitizer that induces photochemical internalization as a result of the activation thereof with a light, and is preferably a sensitizer that generates singlet oxygen as a result of the activation thereof with a light. Examples of the photosensitizing dye used in the present invention may include photosensitizing dyes, which are proposed or used in PCI (Photochemical Internalization) or PDT (Photodynamic Therapy).

Specific examples of the photosensitizer used in the present invention may include Talaporfin Sodium (LASERPHYRIN (registered trademark)), Porfimer Sodium (PHOTOFRIN (registered trademark)), 5-aminolevulinic acid (Levulan (registered trademark)), and 5-amino levulin methyl ester (Metvix (registered trademark)).

Further examples of the photosensitizer used in the present invention may include tetraphenylchlorin-2-sulfonic acid (TPCS2a) and a salt thereof, and disulfonated aluminum phthalocyanine (AlPcS2 and AlPcS2a) and a salt thereof.

Moreover, other examples may also include sulfonated tetraphenylporphyrin (e.g., TPPS2a, TPPS₄, TPPS₁, and TPPS₂ₒ), nile blue, a chlorin derivative, bacteriochlorin, ketochlorin, and natural and synthetic porphyrin.

Further specific examples may include protoporphyrin IX, foscan, chlorin, uroporphyrin I, uroporphyrin III, heptacarboxylporphyrin I, heptacarboxylporphyrin III, hexacarboxylporphyrin I, hexacarboxylporphyrin III, pentacarboxylporphyrin I, pentacarboxylporphyrin III, coproporphyrin I, coproporphyrin III, isocoproporphyrin, harderoporphyrin, isoharderoporphyrin, hematoporphyrin, mesoporphyrin, ethioporphyrin, pyroporphyrin, deuteroporphyrin IX, pemptoporphyrin, ATXs-10, and a 5-aminolevulinic acid derivative.

The photosensitizer used in the present invention may be activated by absorbing a visible light, but it is also preferable for the present sensitizer to absorb a visible light with a long wave length or a near infrared light.

Examples of such a sensitizer may include silicon phthalocyanine, zinc phthalocyanine, and a derivative thereof. Further specific examples may include IR700 (registered trademark) and a derivative thereof.

### < Substance that Binds to Target Substance on Surface of Tumor Cells >

Examples of the substance that binds to a target substance on the surface of tumor cells may include, but are not particularly limited to, an antibody, a ligand, and a peptide.

When an antibody is used as such a substance that binds to a target substance on the surface of tumor cells, it is possible to use an antibody that specifically binds to a target substance on the surface of tumor cells (for example, a protein, such as Epidermal Growth Factor Receptor (EGFR, ERBB 1, ERBB2, ERBB3, or ERBB4), Mesothelin, Ephrin type-A receptor 2 (EphA2), Glypican 3 (GPC3), Cadhelin 17 (CDH17), Cadherin 3 (CDH3), Roundabout homolog 1 (Robo1), or CD20).

The type of the antibody used in the present invention is not particularly limited, and examples of the present antibody may include a mouse antibody, a human antibody, a rat antibody, a rabbit antibody, a sheep antibody, a camel antibody, an avian antibody, and a genetically modified antibody that is artificially modified for the purpose of reducing xenoantigenicity against a human, such as a chimeric antibody or a humanized antibody. Such a genetically modified antibody can be produced by applying a known method. The chimeric antibody is an antibody of the heavy chain and light chain variable regions of a mammalian antibody other than a human antibody, such as a mouse antibody, and the heavy chain and light chain constant regions of a human antibody. The chimeric antibody can be obtained by ligating DNA encoding the variable region of a mouse antibody to DNA encoding the constant region of a human antibody, then incorporating the ligate into an expression vector, and then introducing the expression vector into a host, so that the host is allowed to generate the antibody. The humanized antibody is obtained by transplanting the complementarity determining region (CDR) of a mammalian antibody other than a human antibody, such as a mouse antibody, into the complementarity determining region of a human antibody. A common gene recombination method therefor has been known. Specifically, a DNA sequence designed to ligate the CDR of a mouse antibody to the framework region (FR) of a human antibody is synthesized from several oligonucleotides that have been produced such that they have an overlapping portion at the terminal portions thereof according to a PCR method. The obtained DNA is ligated to DNA encoding the constant region of a human antibody, and the ligate is then incorporated into an expression vector, which is then introduced into a host, so that the host is allowed to generate the antibody (EP 239400, International Publication WO96/02576, etc.).

In addition, a method of obtaining a human antibody has also been known. For example, human lymphocytes are sensitized with a desired antigen or a cell expressing the desired antigen *in vitro,* and then fusing the sensitized lymphocytes with human myeloma cells, such as, for example, U266, so as to obtain a desired human antibody having a binding activity to an antigen (JP Patent Publication (Kokoku) No. 1-59878 B (1989)). Otherwise, a transgenic antibody having all repertoires of human antibody genes is immunized with a desired antigen to obtain a desired human antibody (see WO93/12227, WO92/03918, WO94/02602, WO94/25585, WO96/34096, and WO96/33735). Further, a technique of obtaining a human antibody by panning using a human antibody library has also been known. For example, a human antibody variable region is allowed to express as a single chain antibody (scFv) on the surface of a phage according to a phage display method, and a phage binding to an antigen can be then selected. By analyzing the selected phage gene, a DNA sequence encoding the variable region of a human antibody binding to the antigen can be determined. If the DNA sequence of scFv binding to an antigen is clarified, a suitable expression vector comprising the sequence can be produced, so that a human antibody can be obtained. These methods have already been publicly known, and please refer to WO92/01047, WO92/20791, WO93/06213, WO93/11236, WO93/19172, WO95/01438, and WO95/15388.

The antibody that binds to tumor cells is preferably a humanized or a human antibody, but is not limited thereto.

Moreover, these antibodies may also be low molecular weight antibodies such as antibody fragments, or modified forms of the antibodies, unless they lose the property of recognizing the entire or a part of a protein encoded by an antigen gene present on the surface of tumor cells. The antibody fragment is a part of an antibody that retains a binding ability to surface antigen. Specific examples of the antibody fragment may include Fab, Fab', F(ab')2, Fv, Diabody, and a single chain variable fragment (scFv). In order to obtain such an antibody fragment, a gene encoding such an antibody fragment is constructed, the gene is then introduced into an expression vector, and it may be then expressed in suitable host cells. As a modified form of an antibody, an antibody binding to various types of molecules such as polyethylene glycol (PEG) can also be used.

DNA encoding a monoclonal antibody can be easily isolated and sequenced according to a commonly used method (for example, by using an oligonucleotide probe capable of specifically binding to a gene encoding the heavy chain and light chain of the monoclonal antibody). Hybridoma cells may be preferable starting materials for such DNA. Once such DNA is isolated, it is inserted into an expression vector, and the expression vector is then used to transform host cells such as *E. coli* cells, COS cells, CHO cells, or myeloma cells that do not generate immunoglobulin before they are transformed. Then, a monoclonal antibody can be generated from the transformed host cells.

As a substance that binds to a target substance on the surface of tumor cells, a ligand can be used. When the target substance on the surface of tumor cells is, for example, a receptor such as Epidermal Growth Factor Receptor (EGFR, ERBB 1, ERBB2, ERBB3, or ERBB4), Mesothelin, or Ephrin type-A receptor 2 (EphA2), a ligand against each of the above-described receptors can be used.

As such a substance that binds to a target substance on the surface of tumor cells, a peptide can also be used. A peptide that binds to a target substance on the surface of tumor cells can be designed and produced by those skilled in the art.

### < Cytotoxin >

The cytotoxin is preferably a protein having cytotoxicity, but is not limited thereto. The cytotoxin may also be a compound having a synthetic or natural anticancer action, such as bleomycin, or a compound used in ADC.

Preferred examples of such a protein having cytotoxicity may include saporin, gelonin, Pseudomonas exotoxin, ricin A chain, deglycosylated ricin A chain, a ribosome inactivating protein, alphasarcine, aspergillin, restrictocin, ribonuclease, epipodophyllotoxin, diphtheria toxin, Sliigatoxin, and a mutant or a genetically modified body thereof.

Moreover, an anticancer agent that hardly transfers from the endosome into the cytoplasm can also be used.

### <Conjugate of Substance that Binds to Target Substance on Surface of Tumor Cells and Cytotoxin >

A substance that binds to a target substance on the surface of tumor cells, and a cytotoxin, must bind to each other, directly or indirectly.

When an antibody or a fragment thereof is used as such a substance that binds to a target substance on the surface of tumor cells, as a method of directly chemically binding the antibody or the fragment thereof to a cytotoxin, a binding method used for known ADC can be used. Otherwise, when the cytotoxin is a protein, a bifunctional crosslinking agent can also be used.

Alternatively, when the cytotoxin is a protein, a toxin is fused with an antibody or a fragment thereof by genetic recombination to form a protein, so that an immunotoxin can be produced.

Moreover, as another method, a method of indirectly binding an antibody or a fragment thereof to a cytotoxin by using a second binding pair can also be used. Examples of the second binding pair that can be utilized herein may include avidin-biotin and an antibody-hapten.

Further, in the present invention, it is also possible to use a conjugate of a peptide or a ligand that binds to a target substance on the surface of tumor cells and a toxin, instead of using an immunotoxin in which an antibody and a toxin bind to each other.

### < Administration Methods and Applied Doses >

The method for administering the medicament of the present invention to a subject having a tumor (for example, a cancer, etc.) is not particularly limited.

The conjugate of a substance that binds to a target substance on the surface of tumor cells and a cytotoxin can be administered to the subject, for example, via intravenous administration, arterial administration, intramuscular administration, subcutaneous administration, intradermal administration, intraperitoneal administration, or oral administration.

Alternatively, an administration method involving administration of the conjugate to tumor tissues and the periphery thereof via local injection, application, spraying or the like can also be applied.

The photosensitizing dye can be administered to a subject, for example, via intravenous administration, arterial administration, intramuscular administration, subcutaneous administration, intradermal administration, intraperitoneal administration, or oral administration. Alternatively, an administration method involving administration of the photosensitizing dye to tumor tissues and the periphery thereof via local injection, application, spraying or the like can also be applied.

The applied dose of the conjugate of a substance that binds to a target substance on the surface of tumor cells and a cytotoxin is not particularly limited. The conjugate can be administered to a subject at a dose of, for example, 1 µg/kg of body weight to 100 mg/kg of body weight, and preferably, at a dose of 10 µg/kg of body weight to 10 mg/kg of body weight.

The applied dose of the Talaporfin Sodium or Porfimer Sodium is not particularly limited. The Talaporfin Sodium or Porfimer Sodium can be administered to a subject at a dose of, for example, 1 µg/kg of body weight to 100 mg/kg of body weight, and preferably, at a dose of 10 µg/kg of body weight to 10 mg/kg of body weight.

The number of doses is not particularly limited, and administration can be carried out once to several times (from once to 20 times, and preferably from once to 10 times). Administration can be carried out, for example, every 2 to 4 weeks, or every 1 to 2 months. In addition, the number of light irradiation operations is not particularly limited, either. The light irradiation can be carried out once to several times.

### < Cells and/or Diseases as Targets >

The tumor as a target of the administration of the medicament of the present invention is a tumor that expresses Epidermal Growth Factor Receptor (EGFR, ERBB1, ERBB2, ERBB3, or ERBB4), Mesothelin, Ephrin type-A receptor 2 (EphA2), Glypican 3 (GPC3), Cadhelin 17 (CDH17), Cadhelin 3 (CDH3), Roundabout homolog 1 (Robo1), CD20, or the like on the surface thereof.

Specific examples of the tumor as a target of the administration of the medicament of the present invention may include cancers, such as head and neck cancer, lung cancer, liver cancer, colorectal cancer, skin cancer, esophageal cancer, stomach cancer, cervical cancer, endometrial cancer, mesothelioma, brain tumor, malignant melanoma, breast cancer, bile duct cancer, pancreatic cancer, ovarian cancer, kidney cancer, bladder cancer, prostate cancer, malignant lymphoma, and osteosarcoma.

The following Examples demonstrate that the medicament of the present invention provides effects even on the tumor cell line MCF7, in which the expression level of EGFR is approximately 15,000 molecules per cell, at almost the same level as effects on the tumor cell line A431, in which the expression level of EGFR is approximately 300,000 molecules per cell. Thus, the following Examples demonstrate the present invention.

### [Examples]

### Example 1: Cytotoxicity assay of using aluminum phthalocyanine to MCF7 cells

### < Acquisition of Materials >

As a low EGFR-expressing cell line, MCF7 (human breast cancer cells) was acquired from KAC Company (Kyoto, Japan). As a photosensitizing dye, aluminum phthalocyanine (AlPcS2a) was purchased from Frontier Scientific (State of Utah) and was then used. Cetuximab used as an anti-EGFR antibody and an LED lamp (54 W) having a peak wavelength at 650 nm were purchased from King Do Way (18PCS E27, Amazon.co.jp).

### < Cell Culture >

Using a medium prepared by adding 10% fetal bovine serum and non-essential amino acid to an Eagle's Minimal Essential Medium (EMEM), MCF7 was cultured under conditions of 37°C and 5% CO₂ concentration.

### < Adjustment of Immunotoxin >

Cetuximab dissolved in PBS(-) was mixed with EZ-LINK sulfo-NHS-LC-biotinylation reagent (Thermo Fisher Scientific, Commonwealth of Massachusetts) dissolved in ultrapure water at a molar ratio of 1 : 40, and the obtained mixture was then reacted. Thereafter, the reaction mixture was purified using PD SpinTrap G-25 (GE Healthcare Life Sciences, England). The biotinylated Cetuximab was equivalently mixed with streptavidin-saporin (Biotin-Z Internalization Kit [KIT-27-Z], Advanced Targeting Systems, California), and the obtained mixture was reacted at room temperature for 30 minutes, so as to obtain saporin-bound Cetuximab (IT-Cetuximab).

### < Cytotoxicity Assay >

MCF7 was seeded in a 96-well plate in an amount of 7.5 × 10³ cells per well, and was then cultured overnight. Subsequently, under the following three conditions, addition of an immunotoxin and a photosensitizer and light irradiation were carried out, and the degrees of cytotoxicity under individual conditions were compared with each other.
Condition 1: Addition of only 0.032 - 20 nM IT-Cetuximab
Condition 2: Light irradiation (650 nm, 18.8 mJ/cm²), after addition of 0.032 - 20 nM IT-Cetuximab and 1 µM AlPcS2a

IT-Cetuximab and AlPcS2a were added to the cells under individual conditions, and 16 hours later, the cells were washed once using PBS(-). Thereafter, a novel drug-free medium was added to the resulting cells. Further, 4 hours later, the cell group under Condition 2 was irradiated with a light of 650 nm, so as to result in 18.8 mJ/cm².

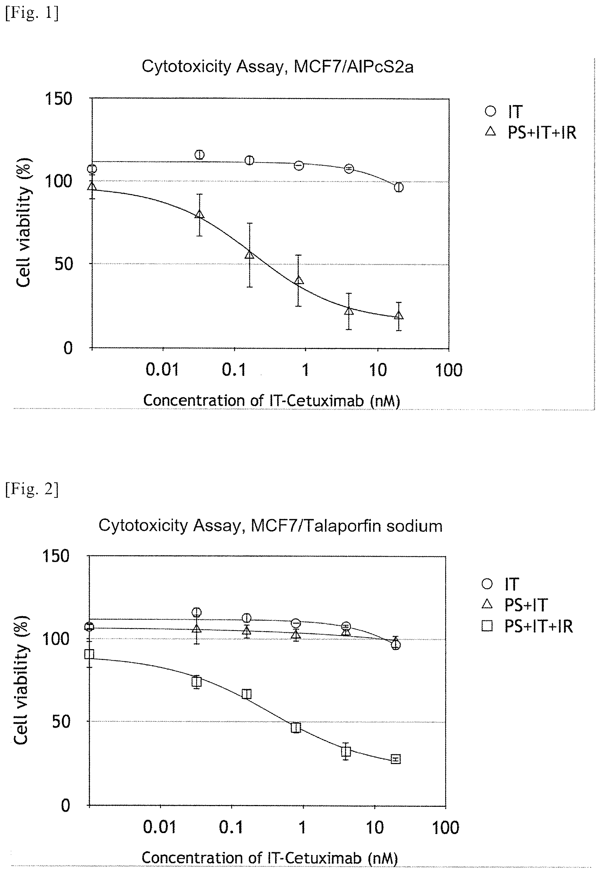

After the cells had been cultured for 72 hours, CCK-8 Kit Solution was added in an amount of 10 µl to each well, and the reaction was then carried out for 1 to 2 hours. Thereafter, the absorption at 450 nm of each well was measured. Based on the absorbance obtained in the same manner as that of Example 1, cell viability (Fig. 2) and the IT-Cetuximab concentration showing 50% viability were calculated (Table 1).

### Example 2: Cytotoxicity assay of using Talaporfin Sodium to MCF7 cells

### < Acquisition of Materials >

As a low EGFR-expressing cell line, MCF7 (human breast cancer cells) was acquired from KAC Company (Kyoto, Japan). With regard to Cetuximab used as an anti-EGFR antibody, Talaporfin Sodium used as a photosensitizing dye, an LED lamp having a peak wavelength at 650 nm, the same products as those used in Comparative Example 1 were used.

### < Cell Culture >

MCF7 was cultured by the same method as that of Example 1.

### < Adjustment of Immunotoxin >

The same saporin-conjugated Cetuximab (IT-Cetuximab) as that produced in Comparative Example 1 was used.

### < Cytotoxicity Assay >

MCF7 was seeded in a 96-well plate in an amount of 7.5 × 10³ cells per well, and was then cultured overnight. Subsequently, under the following three conditions, addition of an immunotoxin and a photosensitizer and light irradiation were carried out, and the degrees of cytotoxicity under individual conditions were compared with one another.
Condition 1: Addition of only 0.032 - 20 nM IT-Cetuximab
Condition 2: Addition of 0.032 - 20 nM IT-Cetuximab and 20 µM Talaporfin Sodium
Condition 3: Light irradiation (650 nm, 37.6 mJ/cm²), after addition of 0.032 - 20 nM IT-Cetuximab and 20 µM Talaporfin Sodium

IT-Cetuximab and Talaporfin Sodium were added to the cells under individual conditions, and 16 hours later, the cells were washed once using PBS(-). Thereafter, a novel drug-free medium was added to the resulting cells. Further, 4 hours later, the cell group under Condition 3 was irradiated with a light of 650 nm, so as to result in 37.6 mJ/cm².

After the cells had been cultured for 72 hours, CCK-8 Kit Solution was added in an amount of 10 µl to each well, and the reaction was then carried out for 1 to 2 hours. Thereafter, the absorption at 450 nm of each well was measured. Based on the absorbance obtained in the same manner as that of Example 1, cell viability (Fig. 3) and the IT-Cetuximab concentration showing 50% viability were calculated (Table 1).

**[Table 1]**

| EC50 of IT-Cetuximab in each example | | | |
|---|---|---|---|
| | Cell Type | Conditions | EC50 |
| Ex. 1 | MCF7 | IT-Cetuximab + AlPcS2a +Light Irradiation | 0.29 nM |
| Ex. 2 | MCF7 | IT-Cetuximab + Talaporfin Sodium + Light Irradiation | 0.61 nM |

The expression level of EGFR as target molecules per cell was approximately 300,000 molecules in A431, whereas it was approximately 15,000 molecules in MCF7 (Non-Patent Documents 2 and 3).
Non-Patent Document 2: Antibody-dependent cellular cytotoxicity mediated by cetuximab against lung cancer cell lines., Kurai J, Chikumi H, Hashimoto K, Yamaguchi K, Yamasaki A, Sako T, Touge H, Makino H, Takata M, Miyata M, Nakamoto M, Burioka N, Shimizu E. Clin Cancer Res 2007, 13(5), 1552-61.
Non-Patent Document 3: ear infrared fluorescence imaging of EGFR expression in vivo using IRDye800CW-nimotuzumab.,Bernhard W1, El-Sayed A1, Barreto K1, Gonzalez C1, Hill Wl, ParadaAC2, Fonge H3,4,5, Geyer CR1., Oncotarget, 2018, 9(5), 6213-6227

Using the photosensitizing dye AlPcS2a, a cytotoxicity experiment was carried out against the MCF7 cell line (Example 1). In the case of MCF7, the expression level of EGFR was low, and even though immunotoxin-bound Cetuximab was added in a concentration of 20 nM to the cells, no cytotoxic effects could be obtained. However, when AlPcS2a was added to the cells simultaneously with IT-Cetuximab, followed by light irradiation, cell internalization efficiency could be improved, and as a result, sufficient cytotoxic effects (EC50 = 0.29 nM) could be obtained.

Using the photosensitizing dye Talaporfin Sodium, a cytotoxicity experiment was carried out against the MCF7 (Example 2). In this case also, it was found that sufficient cytotoxic effects (EC50 = 0.61 nM) could be obtained by adding IT-Cetuximab and Talaporfin Sodium to the MCF7, and then, further performing light irradiation.

From Examples 1 and 2, it was found that tumor cell-damaging activity is expressed even in a low EGFR-expressing cell line.

## Claims

1. A medicament for killing tumor cells that express a target substance at a low level on the cell surface thereof, comprising:
a conjugate of a substance that binds to a target substance on the surface of tumor cells and a cytotoxin; and
a photosensitizing dye.

2. The medicament according to claim 1, wherein the expression level of the target antigen is 20,000 molecules or less per cell.

3. The medicament according to claim 1 or 2, which is a medicament for killing a tumor cell group comprising tumor cells, in which the expression level of the target substance is 20,000 molecules or less per cell.

4. The medicament according to any one of claims 1 to 3, wherein the substance that binds to the target substance on the surface of the tumor cells is an antibody, an antibody fragment, a ligand, or a peptide.

5. The medicament according to any one of claims 1 to 4, wherein the cytotoxin is saporin, gelonin, or Pseudomonas exotoxin.

6. The medicament according to any one of claims 1 to 5, wherein the photosensitizing dye is Talaporfin Sodium, Porfimer Sodium, AlPcS2a, or TPCS2a.

7. The medicament according to any one of claims 1 to 6, wherein the tumor cells are cells that express Epidermal Growth Factor Receptor (EGFR, ERBB1, ERBB2, ERBB3, or ERBB4), Mesothelin, Ephrin type-A receptor 2 (EphA2), Glypican 3 (GPC3), Cadherin 17 (CDH17), or Roundabout homolog 1 (Robo1) on the cell surface thereof.

8. The medicament according to any one of claims 1 to 7, wherein the tumor cells are cancer cells of any one of head and neck cancer, lung cancer, liver cancer, colorectal cancer, skin cancer, esophageal cancer, stomach cancer, cervical cancer, endometrial cancer, mesothelioma, brain tumor, malignant melanoma, breast cancer, bile duct cancer, pancreatic cancer, ovarian cancer, kidney cancer, bladder cancer, prostate cancer, malignant lymphoma, and osteosarcoma.

9. The medicament according to any one of claims 1 to 8, which kills tumor cells by performing the following steps on the cells:
(1) a step of allowing the conjugate to come into contact with tumor cells;
(2) a step of allowing a photosensitizing dye to come into contact with the tumor cells; and
(3) a step of irradiating the tumor cells with a wavelength effective for activating the photosensitizing dye, so as to kill the cells.

10. The medicament according to any one of claims 1 to 9, which kills tumor cells by performing the following steps on the cells:
(1) a step of allowing the conjugate and a photosensitizing dye to come into contact with tumor cells; and then,
(2) a step of irradiating the tumor cells with a wavelength effective for activating the photosensitizing dye, so as to kill the cells.
